# EUROPÄISCHE PATENTSCHRIFT

(11) **EP 0 518 189 B1**
(45) Veröffentlichungstag und Bekanntmachung des Hinweises auf die Patenterteilung: **08.07.1998**
(21) Anmeldenummer: 92109322.5
(22) Anmeldetag: 03.06.1992
(51) Int. Cl.: C07D 213/30, C09K 19/34, G02F 1/13

(54) **Phenylpyridine**
Phenyl pyridins
Phényl pyridines

(30) Priorität: 14.06.1991 CH 1772/91
(43) Veröffentlichungstag der Anmeldung: 16.12.1992
(73) Patentinhaber: Rolic AG, 6301 Zug (CH)
(72) Erfinder: Fünfschilling, Jürg, CH-4054 Basle (CH); Kelly, Stephen, CH-4313 Möhlin (CH); Villiger, Alois, CH-4055 Basle (CH)
(74) Vertreter: Eder, Carl E.

(56) Entgegenhaltungen:
- EP-A- 0 401 522
- ADVANCES IN LIQUID CRYSTAL RESEARCH AND APPLICATIONS,Proc. Liq. Cryst. Conf. Soc. Countries,3rd 1979 (Pub.1981),vol.2,pages 1007-13,PERGAMON, OXFORD, GB A. I. PAVLUCHENKO: *Liquid crystalline pyridine derivatives*

## Beschreibung

Die vorliegende Erfindung betrifft Phenylpyridin-Derivate, flüssigkristalline Gemische, die solche Verbindungen enthalten, sowie deren Verwendung für elektro-optische Zwecke.

Flüssige Kristalle werden vor allem als Dielektrika in Anzeigevorrichtungen eingesetzt, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Beispiele solcher Vorrichtungen sind Zellen mit dynamischer Streuung, DAP-Zellen (Deformation aufgerichteter Phasen), SSF-Zellen (surface stabilized ferroelectric), DHF-Zellen (deformed helix ferroelectric) oder SBF-Zellen (short-pitch bistable ferroelectric).

Die Flüssigkristallmaterialien müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten und einen hohen Kontrast ergeben. Weiterhin sollten sie bei üblichen Betriebstemperaturen von etwa -30°C bis etwa +80°C, insbesondere von etwa -20°C bis etwa +60°C eine geeignete Mesophase besitzen, beispielsweise für die oben genannten Zellen eine breite smektische Mesophase.

Da Flüssigkristalle in der Regel als Mischungen mehrerer Komponenten zur Anwendung gelangen, ist es wichtig, dass die Komponenten untereinander gut mischbar sind.

Gegenstand der vorliegenden Erfindung sind die Verbindungen der allgemeinen Formel 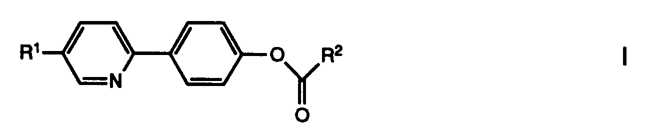 worin R¹ eine geradkettige Alkylgruppe mit 7 bis 9 Kohlenstoffatomen bedeutet; und R² eine geradkettige Alkylgruppe mit 6 bis 11 Kohlenstoffatomen darstellt.

Es wurde überraschenderweise gefunden, dass die erfindungsgemässen Verbindungen für ferroelektrische Anwendungen ausserordentlich günstige Eigenschaften besitzen. Obwohl die erfindungsgemässen Verbindungen selber oft keine S_{C} Phase (smektisch C Phase) aufweisen, wird durch ihre Zumischung zu bekannten Grundkomponenten für ferroelektrische Mischungen eine bedeutende Erhöhung des S_{C}-S_{A} oder S_{C}-N Phasenüberganges und eine Verschmälerung, oft sogar eine vollständige Unterdrückung der S_{A} Phase bewirkt; ausserdem wird der Schmelzpunkt signifikant abgesenkt, was zu einer vergleichsweise breiten Mesophase führt. Die erfindungsgemässen Verbindungen weisen zudem breite smektische Phasen mit erstaunlich niedriger Viskosität auf, was zu schnellen Schaltzeiten führt.

Der Ausdruck "geradkettige Alkylgruppe mit 7 bis 9 Kohlenstoffatomen" bedeutet die Gruppen Heptyl, Octyl und Nonyl. Der Ausdruck "geradkettige Alkylgruppe mit 6 bis 11 Kohlenstoffatomen" bedeutet die Gruppen Hexyl, Heptyl, Octyl, Nonyl, Decyl und Undecyl.

Bevorzugt werden Verbindungen der Formel I, worin R¹ Octyl bedeutet. Besonders bevorzugt sind zudem diejenigen Verbindungen der Formel I, worin R² Heptyl, Octyl oder Nonyl bedeutet. Es sind dies die Verbindungen:
4-(5-Octyl-2-pyridyl)phenyl-octansäureester
4-(5-Octyl-2-pyridyl)phenyl-nonansäureester
4-(5-Octyl-2-pyridyl)phenyl-decansäureester.

Diese Verbindungen zeichnen sich durch einen besonders breiten smektischen Mesophasenbereich aus.

Die erfindungsgemässen Verbindungen der Formel I können in an sich bekannter Weise aus 4-(5-Alkyl-2-pyridyl)phenol und einer Fettsäure hergestellt werden. Die Umsetzung kann in Gegenwart von N,N'-Dicyclohexylcarbodiimid und 4-(Dimethylamino)pyridin in z.B. Dichlormethan oder einem anderen geeigneten Lösungsmittel wie z.B. Chloroform erfolgen. Die als Ausgangsmaterialien verwendeten Verbindungen sind bekannt und zum Teil im Handel erhältlich.

Die Verbindungen der Formel I können in Form von Gemischen untereinander und/oder mit anderen Flüssigkristallkomponenten verwendet werden. Die Verbindungen der Formel I werden vorzugsweise in Gemischen mit anderen Flüssigkristallkomponenten verwendet.

Die erfindungsgemässen, flüssigkristallinen Gemische enthalten mindestens 2 Komponenten, wovon mindestens eine Komponente eine Verbindung der Formel I ist. Eine zweite und gegebenenfalls weitere Komponenten können weitere Verbindungen der Formel I sein, wobei jedoch auch mindestens ein chiraler Dotierstoff im Gemisch enthalten sein muss. Derartige weitere Flüssigkristallkomponenten sind vorzugsweise achirale Verbindungen der Formeln 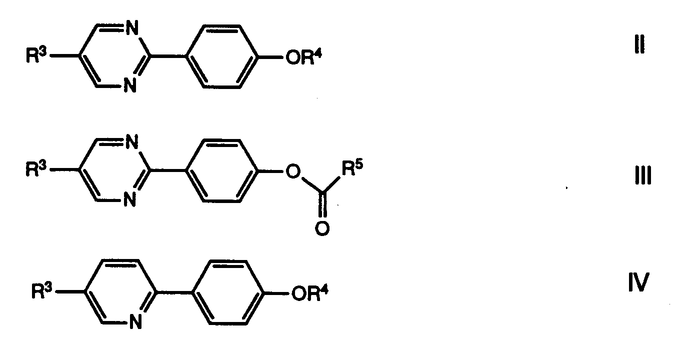 Als Dotierstoffe können Verbindungen der folgenden allgemeinen Formeln genannt werden: 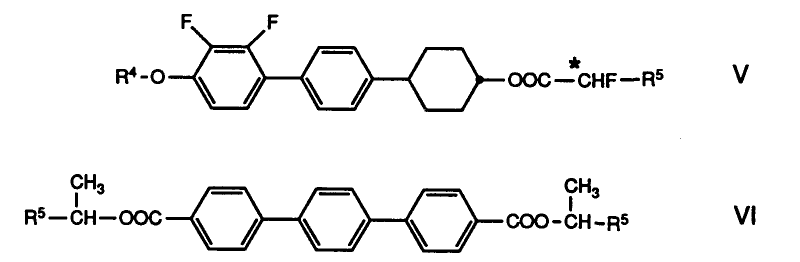 worin R³ Alkyl, Alkoxy, 3E-Alkenyl oder 4-Alkenyl bezeichnet; R⁴ Alkyl oder Alkenyl bedeutet; und R⁵ Alkyl bedeutet.

Der Ausdruck "Alkyl" umfasst unverzweigte oder verzweigte Alkylgruppen mit 1 bis 15 Kohlenstoffatomen, vorzugsweise unverzweigte Alkylgruppen mit 1 bis 12 Kohlenstoffatomen wie Methyl, Aethyl, Propyl, Butyl, Pentyl, Hexyl, Heptyl, Octyl, Nonyl, Decyl, Undecyl und Dodecyl.

Der Ausdruck "Alkoxy" umfasst Gruppen in denen der Alkylrest wie oben definiert ist.

Der Ausdruck "Alkenyl" umfasst Alkenylgruppen mit 2 bis 15 Kohlenstoffatomen wie 2E-Alkenyl, 3Z-Alkenyl, 4-Alkenyl und Alkenyle mit endständiger Doppelbindung.

Die Ausdrücke "2E-Alkenyl", "3E-Alkenyl", "3Z-Alkenyl" und "4-Alkenyl" umfassen unverzweigte Alkenylgruppen mit 3 bis 15, 4 bis 15 bzw. 5 bis 15 Kohlenstoffatomen, in welchen die Doppelbindung in 2,3 bzw. 4-Stellung steht, wobei E und Z die Konfiguration der Doppelbindung bezeichnen. Solche Gruppen sind beispielsweise Allyl, 2E-Butenyl, 2E-Pentenyl, 2E-Hexenyl, 2E-Heptenyl, 2E-Octenyl, 2E-Nonenyl, 2E-Decenyl, 3-Butenyl, 3E-bzw. 3Z-Pentenyl, 3E-bzw. 3Z-Hexenyl, 3E-bzw. 3Z-Heptenyl, 3E-bzw. 3Z-Octenyl, 3E-bzw. 3Z-Nonenyl, 3E-bzw. 3Z-Decenyl, 4-Pentenyl, 4-Hexenyl, 4-Heptenyl, 4-Octenyl, 4-Nonenyl, 4-Decenyl und dergleichen. Der Ausdruck "Alkenyl" mit endständiger Doppelbindung umfasst unverzweigte Alkenyle mit 3 bis 15 Kohlenstoffatomen wie beispielsweise Allyl, 3-Butenyl, 4-Pentenyl, 5-Hexenyl, 6-Heptenyl, 7-Octenyl, 8-Nonenyl, 9-Decenyl und dergleichen.

Aufgrund der guten Löslichkeit der Verbindungen der Formel I und aufgrund ihrer guten Mischbarkeit untereinander kann der Anteil an Verbindungen der Formel I in den erfindungsgemässen Gemischen relativ hoch sein und bis 85 Gew.-% betragen. Bevorzugt wird im allgemeinen ein Anteil von Verbindungen der Formel I von etwa 1-50 Gew.-%, inbesondere von etwa 5-35 Gew.-%.

Die Herstellung der flüssigkristallinen Gemische und der elektrooptischen Vorrichtungen kann in an sich bekannter Weise erfolgen.

Die Erfindung wird durch die folgenden Beispiele weiter veranschaulicht. In den Beispielen bedeutet C eine kristalline, N eine nematische, S eine smektische und I die isotrope Phase. Die Phasenübergangstemperaturen werden jeweils als N-I (in diesem Fall Uebergang von der nematischen Phase in den isotropen Zustand) bezeichnet.

### Beispiel 1

0,5 g 4-(5-Octyl-2-pyridyl)phenol, 0,2 g Heptansäure und 0,04g 4-(Dimethylamino)pyridin wurden in 50 ml Dichlormethan gelöst und die Lösung innert 10 Minuten unter Rühren portionenweise mit 0,4 g N,N'-Dicyclohexylcarbodiimid versetzt. Das Gemisch wurde über Nacht bei Raumtemperatur gerührt und dann filtriert. Das Filtrat wurde mit Dichlormethan verdünnt, zweimal mit je 50 ml gesättigter Natriumcarbonat-Lösung und dann mit Wasser gewaschen, über Magnesiumsulfat getrocknet, filtriert und anschliessend eingeengt. Das erhaltene Rohprodukt wurde an Kieselgel mit Toluol chromatographisch gereinigt. Umkristallisation aus Aethanol ergab 0,4 g 4-(5-Octyl-2-pyridyl)phenyl-heptansäureester, mit einem Smp. (C-S) 57°C und Klp. (S-I) 77°C.

Auf analoger Weise wurden folgende Verbindungen hergestellt:
4-(5-Heptyl-2-pyridyl)phenyl-heptansäureester, Smp. (C-S₄) 48°C, (S₄-S_{F}) 68°C, Klp. (S_{F}-I) 74°C;
4-(5-Heptyl-2-pyridyl)phenyl-octansäureester, Smp. (C-S₄) 50°C, (S₄-S_{F}) 68°C, (S_{F}-S_{C}) 76°C, Klp. (S_{C}-I) 77°C;
4-(5-Heptyl-2-pyridyl)phenyl-nonansäureester, Smp. (S-S₄) 52°C, (S₄-S_{F}) 68°C, (S_{F}-S_{C}) 77,5°C, Klp. (S_{C}-I) 78,5°C;
4-(5-Heptyl-2-pyridyl)phenyl-decansäureester, Smp. (S-S₄) 56°C, (S₄-S_{F}) 69°C, (S_{F}-S_{C}) 78,5°C, Klp. (S_{C}-I) 81°C;
4-(5-Heptyl-2-pyridyl)phenyl-undecansäureester, Smp. (C-S₄) 52°C, (S₄-S_{F}) 66°C, (S_{F}-S_{C}) 79°C, Klp. (S_{C}-I) 81°C;
4-(5-Heptyl-2-pyridyl)phenyl-dodecansäureester, Smp. (C-S₄) 54°C, (S₄-S_{F}) 68°C, (S_{F}-S_{C}) 79°C, Klp. (S_{C}-I) 82°C;
4-(5-Octyl-2-pyridyl)phenyl-octansäureester, Smp. (C -S) 60°C und Klp. (S-I) 80°C,
4-(5-Octyl-2-pyridyl)phenyl-nonansäureester, Smp. (C -S) 61°C und Klp. (S-I) 81°C,
4-(5-Octyl-2-pyridyl)phenyl-decansäureester, Smp. (C -S) 61°C und Klp. (S-I) 83°C,
4-(5-Octyl-2-pyridyl)phenyl-undecansäureester, Smp. (C-S) 42°C und Klp. (S-I) 83°C,
4-(5-Octyl-2-pyridyl)phenyl-dodecansäureester, Smp. (C-S) 51°C und Klp. (S-I) 83°C,
4-(5-Nonyl-2-pyridyl)phenyl-heptansäureester, Smp. (C-S_{C} 60°C, Klp. (S_{C}-I) 83°C;
4-(5-Nonyl-2-pyridyl)phenyl-octansäureester, Smp. (C-S_{C}) 66°C, Klp. (S_{C}-I) 86°C;
4-(5-Nonyl-2-pyridyl)phenyl-nonansäureester, Smp. (C-S_{C}) 70°C, Klp. (S_{C}-I) 87°C;
4-(5-Nonyl-2-pyridyl)phenyl-decansäureester, Smp. (C-S_{C}) 71°C, Klp. (S_{C}-I) 89°C;
4-(5-Nonyl-2-pyridyl)phenyl-undecansäureester, Smp. (C-S_{C}) 66°C, Klp. (S_{C}-I) 89°C;
4-(5-Nonyl-2-pyridyl)phenyl-dodecansäureester, Smp. (C-S_{C}) 65°C, Klp. (S_{C}-I) 90°C.

### Beispiel 2

Zur Untersuchung der Eigenschaften der Verbindungen der Formel I wurde eine Grundmischung hergestellt und jeweils 15% einer Verbindung der Formel I zugemischt. Die Phasenübergangstemperaturen dieser Mischungen wurden bestimmt; die Kristallisationstemperatur T_{C} wurde dabei aus Leitfähigkeitsdaten bestimmt. Die Schaltzeiten wurden bei 25°C (10 Vpp/µ, Zeit vom Start des Pulses bis zum Maximum des Stromes) gemessen. Die Messwerte sind in der Tabelle 1 zusammengefasst.

### Grundmischung

- 16 Gew.-%: p-[trans-4-{[(R)-2-Fluorhexanoyl]oxy}cyclohexyl]phenyl-2,3-difluor-4-(octyloxy)benzoesäureester;
- 24 Gew.-%: 2-[p-(Hexyloxy)phenyl]-5-nonylpyrimidin;
- 24 Gew.-%: 2-[p-(Nonyloxy)phenyl]-5-nonylpyrimidin;
- 12 Gew.-%: 2-[p-(Nonyloxy)phenyl]-5-heptylpyrimidin;
- 12 Gew.-%: 2-[p-(Heptyloxy)phenyl]-5-octylpyrimidin;
- 12 Gew.-%: 2-[p-(Decyloxy)phenyl]-5-octylpyrimidin.

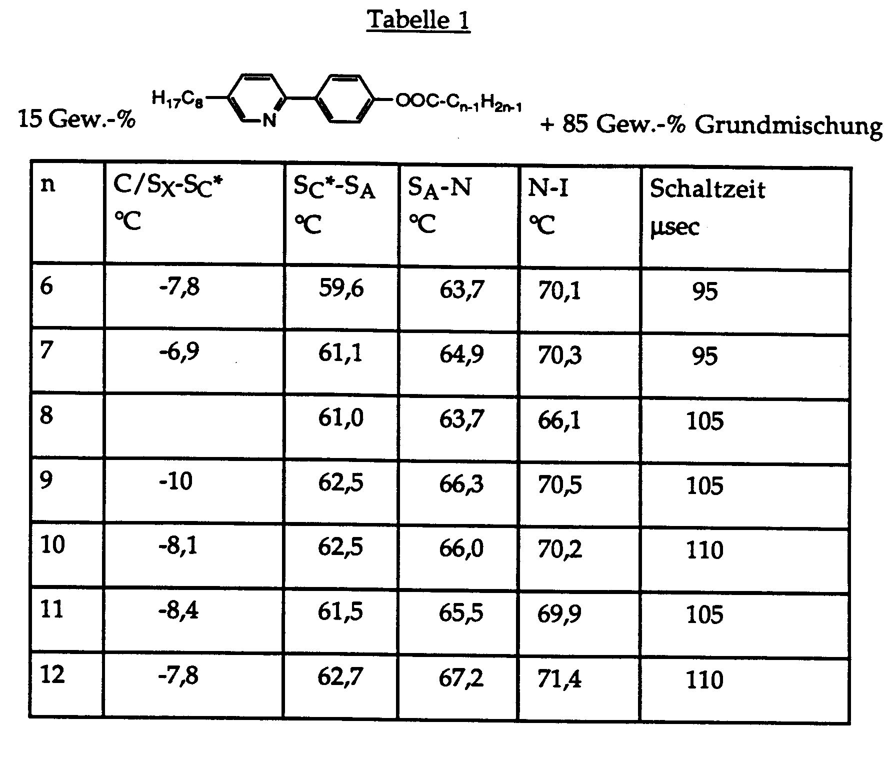

### Beispiel 3

Die Daten für zwei Mischungen für SBF-Anwendungen, die Vertreter der erfindungsgemässen Verbindungen enthalten, sind in Tabelle 2 zusammengetragen.

### Grundmischung

- 43 Gew.-%: Bis[(S)-1-methylheptyl]-p-terphenyl 4,4'-dicarbonsäureester
- 28 Gew.-%: 2-[4-(Hexyloxy)phenyl]-5-nonylpyrimidin;
- 29 Gew.-%: 2-[4-(Nonyloxy)phenyl]-5-nonylpyrimidin;

### Mischung I

- 16,5 Gew.-%: 4-(5-Octyl-2-pyridyl)phenyl heptansäureester
- 22,5 Gew.-%: 4-(5-Octyl-2-pyridyl)phenyl octansäureester
- 61 Gew.-%: Grundmischung

### Mischung II

- 21,5 Gew.-%: 4-(5-Nonyl-2-pyrimidinyl)phenyl nonansäureester
- 18 Gew.-%: 4-(5-Nonyl-2-pyrimidinyl)phenyl decansäureester
- 60,5 Gew.-%: Grundmischung

**Tabelle 2**

| Mischung | C/Sχ-S_{C}* °C | S_{C}*-S_{A} °C | S_{A}-I °C | S_{C}-I °C | Schaltzeit µsec |
|---|---|---|---|---|---|
| I | <-9 | | | 65,7 | 42 |
| II | >0 | 58,0 | 61,0 | | 42 |

## Patentansprüche

1. Verbindungen der allgemeinen Formel 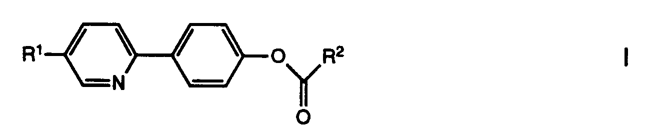 worin R¹ eine geradkettige Alkylgruppe mit 7 bis 9 Kohlenstoffatomen darstellt und R² eine geradkettige Alkylgruppe mit 6 bis 11 Kohlenstoffatomen darstellt.

2. Verbindungen nach Anspruch 1, dadurch gekennzeichnet, dass R¹ Octyl bedeutet.

3. Verbindungen gemäss einem der Ansprüche 1 oder 2, dadurch gekennzeichnet, dass R² Heptyl, Octyl oder Nonyl bedeutet.

4. Verbindung gemäss einem der Ansprüche 1 bis 3, dadurch gekennzeichnet, dass R¹ Octyl und R² Heptyl bedeuten.

5. Verbindung gemäss einem der Ansprüche 1 bis 3, dadurch gekennzeichnet, dass R¹ Octyl und R² Octyl bedeuten.

6. Verbindung gemäss einem der Ansprüche 1 bis 3, dadurch gekennzeichnet, dass R¹ Octyl und R² Nonyl bedeuten.

7. Flüssigkristallines Gemisch mit mindestens zwei Komponennten, dadurch gekennzeichnet, dass mindestens eine Komponente eine Verbindung der in Anspruch 1 definierten Formel I ist.

8. Verwendung von Verbindungen der in Anspruch 1 definierten Formel I für elektro-optische Zwecke.

## Claims

1. Compounds of the general formula 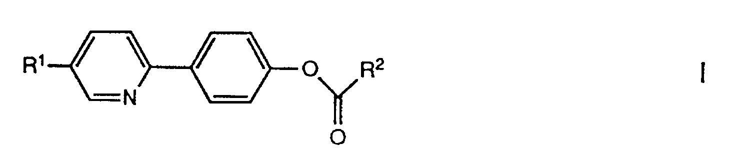 characterized in that R¹ signifies a straight-chain alkyl group with 7 to 9 carbon atoms and R² represents a straight-chain alkyl group with 6 to 11 carbon atoms.

2. Compounds according to claim 1 characterized in that R¹ signifies octyl.

3. Compounds according to any one of claims 1 or 2, characterized in that R² signifies heptyl, octyl or nonyl.

4. The compound according to any one of claims 1 to 3, characterized in that R¹ signifies octyl and R² signifies heptyl.

5. The compound according to any one of claims 1 to 3, characterized in that R¹ signifies octyl and R² signifies octyl.

6. The compound according to any one of claims 1 to 3, characterized in that R¹ signifies octyl and R² signifies nonyl.

7. A liquid crystalline mixture with at least two components, characterized in that at least one component is a compound of formula I defined in claim 1.

8. The use of compounds of formula I defined in claim 1 for electro-optical purposes.

## Revendications

1. Composés de formule générale 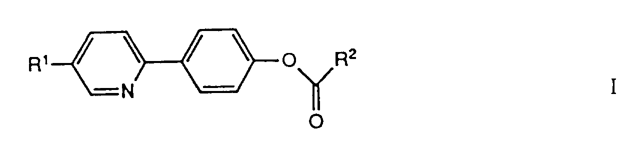 où R¹ représente un groupe alkyle à chaîne linéaire comportant 7 à 9 atomes de carbone et R² représente un groupe alkyle à chaîne linéaire comportant 6 à 11 atomes de carbone.

2. Composés selon la revendication 1, caractérisés en ce que R¹ est le groupe octyle.

3. Composés selon la revendication 1 ou 2, caractérisés en ce que R² est le groupe heptyle, octyle ou nonyle.

4. Composés selon l'une des revendications 1 à 3, caractérisé en ce que R¹ est le groupe octyle et R² est le groupe heptyle.

5. Composés selon l'une des revendications 1 à 3, caractérisé en ce que R¹ est le groupe octyle et R² est le groupe octyle.

6. Composés selon l'une des revendications 1 à 3, caractérisé en ce que R¹ est le groupe octyle et R² est le groupe nonyle.

7. Mélange de cristaux liquides compostant au moins un composant est un composé de formule I définie à la revendication 1.

8. Utilisation de composés de formule I définie à la revendication 1 à des fins d'optique électronique.
